# EUROPEAN PATENT APPLICATION

(11) **EP 4 678 174 A1**
(43) Date of publication of application: **14.01.2026**
(21) Application number: 24382745.8
(22) Date of filing: 11.07.2024
(51) Int. Cl.: A61K 31/44, A61P 3/00, A61P 25/28

(54) **NIZUBAGLUSTAT FOR USE IN THE TREATMENT OF LYSOSOMAL STORAGE DISORDERS**

(71) Applicant: Azafaros B.V., 2333 CH Leiden (NL)
(72) Inventor: LANDSKRONER, Kyle, 2333 CH Leiden (NL); FREITAG, Christian, 2333 CH Leiden (NL); LOPEZ DE FRUTOS, Laura, 2333 CH Leiden (NL)
(74) Representative: Boult Wade Tennant LLP

(57) **Abstract**

The invention relates to nizubaglustat for use in the treatment of a lysosomal storage disorder in a human subject. Specifically, the invention relates to the administration of nizubaglustat at a dose of 3mg - 9mg total/day.

## Description

### FIELD

The invention relates to the use of nizubaglustat in treating a lysosomal storage disorder in a human subject. Specifically, the invention relates to the use of nizubaglustat at a dose of 3mg - 9mg total/day.

### BACKGROUND

Lysosomal storage diseases or disorders (LSDs) are inborn errors of metabolism characterized by the accumulation of substrates in excess in various organs' cells due to the defective functioning of lysosomes. They cause dysfunction of those organs where they accumulate and contribute to great morbidity and mortality. Although rare individually, their prevalence is significant when viewed collectively.

LSDs are diseases typically caused by defects in single genes. Enzyme defects cause nearly seventy percent of the LSDs, and the rest are defects in enzyme activator or associated proteins. Seventy LSDs are described so far, and many more will likely be discovered in the future.

Amongst the different LSDs are GM2 gangliosidosis (e.g. Tay Sachs disease, Sandhoff disease and GM2 activator deficiency), Niemann-Pick disease A, B, and C (transmembrane protein defect C1, soluble non-enzymatic protein defect C2), Gaucher disease types 1, 2, and 3, Pompe disease (glycogen storage disease type II), Fabry disease (classic and late-onset types), metachromatic leukodystrophy, Globoid leukodystrophy (Krabbe disease), and GM1 gangliosidosis types 1, 2, and 3.

GM2 gangliosidoses are a group of degenerative/inflammatory diseases affecting the brain caused by a deficiency of different proteins needed to break down lipids. Examples of a GM2 gangliosidosis include Sandhoff disease and Tay-Sachs disease. GM2 gangliosidoses are characterized by rapid neurological deterioration and typically premature death since treatment success has been limited to date. They are a result of the inheritance of defects in genes responsible for encoding the enzymes required to breakdown GM2 ganglioside (a lipid). This lipid then accumulates in the body, reaching its highest levels in the brain where it is most abundant. In their severe infantile form, these diseases become evident at about 6 months of age. Juvenile- and adult-onset forms of the diseases are associated with a wide range of neurological symptoms, many of which are debilitating and ultimately lethal.

GM2 ganglioside is normally degraded in a cell's lysosomes through the concerted action of the products of three genes, *HEXA*, *HEXB*, and *GM2A.* A defect in any one of these genes can result in a deficiency of *HEXA* activity towards GM2 ganglioside, which then cannot be broken down. The most common form of GM2 gangliosidosis is Tay-Sachs disease, which results from pathogenic variants in *HEXA.* Pathogenic variants in *HEXB*, encoding the beta subunit of β-hexosaminidase, result in Sandhoff disease. Approximately 1 in 25 individuals of Ashkenazi Jewish descent is a carrier for Tay-Sachs disease, but carrier screening has successfully reduced the incidence in that population. The prevalence of Sandhoff disease is approximately 1 in 384,000 live births, but is increased in certain populations.

Niemann-Pick disease is a group of rare conditions which affect the body's ability to break down and use fats, such as cholesterol and lipids, inside cells. Because of the buildup of fats, these cells do not work as they should and, over time, the cells die. Niemann-Pick disease can affect the brain, nerves, liver, spleen and bone marrow. Sometimes it can affect the lungs. Symptoms of Niemann-Pick disease relate to worsening function of the nerves, brain and other organs over time. The symptoms include loss of muscle control, muscle weakness, stiff movements, vision problems, hearing loss, being sensitive to touch, problems sleeping, problems swallowing and eating, slurred speech, mental health conditions, liver and spleen enlargement, and repeated infections.

Niemann-Pick disease can happen at different ages but mainly affects children. Some infants with type A show symptoms within the first few months of life. Those with type B may not show symptoms for years and have a better chance of living to adulthood. People with type C can start to have symptoms at any age but may not have any symptoms until adulthood. The condition has no known cure and is sometimes fatal. Treatment is focused on helping people live with their symptoms.

Miglustat (N-butyldeoxynojirimycin), which is an iminosugar (a synthetic analogue of D-glucose) that was developed as an inhibitor of glucosylceramide synthase (also called ceramide glucosyltransferase, EC 2.4.1.80, UniProt code: Q16739) exhibits a large volume of distribution and has the capacity to access deep organs such as the brain, bone and lung. Currently, miglustat is used to treat Gaucher disease type 1, and, in combination with cipaglucosidase alfa, to treat Pompe disease. In a limited number of countries, miglustat is approved for the treatment of Niemann-Pick Type C disease. Miglustat has not shown to be therapeutically effective for juvenile GM2 gangliosidosis (Maegawa et al. (2009), Molecular Genetics and Metabolism: 98, 215-224). Miglustat is associated with different side effects, including diarrhoea, stomach pain or bloating, gas, loss of appetite, weight loss, upset stomach, vomiting, constipation, dry mouth, weakness, muscle cramps, back pain, dizziness, headache, memory problems, tiredness, or fever.

WO2015/147639 A1 describes a wide range of derivatives of deoxynojirimycin, including nizubaglustat which has formula (I), as shown below (PubChem CID: 101882900, CAS Number: 1633666-49-5).

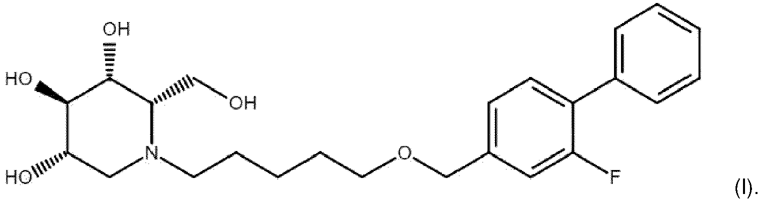

Nizubaglustat is a potent dual inhibitor of glucosylceramide synthase (GCS) and non-lysosomal glucosylceramidase (NLGase, GBA2, UniProt code: Q9HCG7), and is currently undergoing Phase II study for treating GM2 gangliosidosis and Niemann-Pick disease type C.

WO2022/069709 describes crystalline forms of nizubaglustat. It also describes the use of nizubaglustat in the mice model of Niemann-Pick disease type C.

WO2023/131720 describes the use of nizubaglustat in the mice model of Sandhoff disease.

Although the effects of nizubaglustat on various lysosomal storage disorders have been studied in animal models, a need exists for establishing a safe and effective dose of nizubaglustat in human patients suffering from a lysosomal storage disorder.

### SUMMARY OF THE INVENTION

The inventors of the present application analysed clinical and pre-clinical data and found optimal doses of nizubaglustat for treating human subjects in a safe and efficacious manner. The doses were tested in phase II clinical trials on subjects suffering from lysosomal storage disorders, such as GM2 gangliosidosis (e.g. Sandhoff disease) or Niemann-Pick disease type C and were found to deliver therapeutic effects while causing no serious or severe treatment-emergent adverse reactions in the subjects.

Lasting functional improvement (e.g. ability to move, or speak clearly) in patients suffering from progressive or degenerative disorders, such as disorders related to deficiencies in lysosomal storage, is rare, and if it occurs, it is first observed after a prolonged (e.g. 6-12 months) administration of the active. Surprisingly, nizubaglustat at the optimal dose(s) was able to deliver a significant improvement in patients' Scale for the Assessment and Rating of Ataxia (SARA) scores within 4 weeks of first administration. Importantly, the improvement was maintained (or further improved) throughout the duration of the study (about 54 weeks). Similarly, administration of nizubaglustat significantly reduced the plasma concentration of glucosylceramide (GlcCer) C16 and GlcCer C18 - this reduced concentration of GlcCer C16 and GlcCer C18 was maintained throughout the duration of the study.

Unexpectedly, the optimal dose of nizubaglustat also allowed to reduce the frequency and/or duration of seizures in lysosomal storage disorder patients suffering from seizures.

Thus, in a first aspect, the invention provides nizubaglustat for use in treating a lysosomal storage disorder in a human subject suffering from the lysosomal storage disorder, wherein nizubaglustat is administered to the subject at a dose of between about 3mg - about 9mg total/day.

The lysosomal storage disorder may be GM2 gangliosidosis (e.g. Sandhoff disease) and/or Niemann-Pick disease (e.g. type C).

In a second aspect, the invention provides nizubaglustat for use in reducing the duration and/or frequency of seizures in a human subject suffering from seizures, wherein nizubaglustat is administered to the subject at a dose of between about 3mg - about 9mg total/day.

Preferably, the subject also suffers from a lysosomal storage disorder.

In a further aspect, the invention provides a pharmaceutical composition comprising nizubaglustat at a dose of between about 3mg - about 9mg and at least one pharmaceutically acceptable carrier.

In all aspects and embodiments described herein, preferably, the dose of nizubaglustat is about 9mg total/day (e.g. 9 mg total/day).

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

### BRIEF DESCRIPTION OF THE DRAWINGS

These and other aspects of the invention will now be described with reference to the accompanying Figures, in which:
Figure 1 shows the phase 2 study protocol for using nizubaglustat to treat lysosomal storage disorders, such as GM2 gangliosidosis (GM2) or Niemann-Pick disease type C (NPC).
Figure 2 illustrates the pharmacokinetic data, which show measurable concentrations of nizubaglustat across all patients dosed. In the figure, the nizubaglustat concentrations were measured after single dose (day 1 of administration - Visit 1) and after repeat administration for 4 weeks (Visit 2).
Figure 3 illustrates the glucosyl (beta) ceramide (GlcCer) C16 plasma level reductions across NPC and GM2 patients at the 3 mg and 9 mg doses (and placebo) over the period of 12 weeks of administration of nizubaglustat (or placebo).
Figure 4 illustrates the summary of GlcCer C16 and GlcCer C18 plasma levels in the GM2 and NPC patients (combined) tested at 4 weeks and 12 weeks post-nizubaglustat administration (either 9 mg or 3 mg dose).
Figure 5 illustrates the summary of GlcCer C16 and GlcCer C18 plasma levels in the GM2 and NPC patients (split) tested at 4 weeks and 12 weeks post-nizubaglustat administration (either 9 mg or 3 mg dose). The figure includes placebo data.
Figure 6 illustrates the Scale for the Assessment and Rating of Ataxia (SARA) data for phase 2 study.
Figure 7 shows the SARA data at different time points in the study.
Figure 8 shows a plot of the correlation between the maximal % reduction in the plasma GlcCer (at any time point) vs. the exposure (AUC) following the first visit (visit 1).
Figure 9 illustrates the type and frequency of seizures experienced by some of the patients before enrolling in the phase 2 study.
Figure 10 illustrates the duration of seizures experienced by the patients taking part in the phase 2 study.

### DETAILED DESCRIPTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a dose of about 9 mg total/day means a dose of between 8.1 mg - 9.9 mg total/day. For example, a range of between about 3 mg - about 9 mg total/day means a range of between 2.7 mg - 9.9 mg total/day.

The term "optimal dose" (or "the dose") as used herein is intended to refer to a dose of nizubaglustat of between about 3 mg - about 9 mg total/day. In the preferred embodiments, the optimal dose is about 9 mg total/day, for example 9 mg total/day. The optimal dose is understood to deliver clinically significant effect (e.g. lessening of the symptoms of the disease) without causing any serious or severe adverse reactions in a human subject. Typically, the optimal dose administered to the human subject results in a blood concentration of nizubaglustat of between 12-400 ng/mL after administration, preferably between 25-240 ng/mL after administration. Concentrations of nizubaglustat may also be measured from urine and CSF samples. The concentration of nizubaglustat may be measured by Liquid Chromatography with tandem mass spectrometry (LC-MS-MS). The term "after administration" refers to a time point at least 2 hours, at least 4 hours, at least 6 hours, or at least 12 hours after the administration of nizubaglustat. For example, in the context of measuring the concentration of nizubaglustat from blood samples, the samples may be collected between 2-6 hours after administration of nizubaglustat. For example, in the context of measuring the concentration of nizubaglustat from CSF samples, the samples may be collected between 1-12 (e.g. 1-2) hours after administration of nizubaglustat. The optimal dose may also be understood as the "therapeutically effective amount"; i.e. the amount of nizubaglustat that, when administered to a patient for treating a disease, is sufficient to effect such treatment for the disease. The terms "therapeutically effective amount" and "amount effective to" deliver the effect intend to encompass the amount of nizubaglustat (or the pharmaceutical composition comprising nizubaglustat) that, when administered to a patient for delivering a therapeutic effect (e.g. treating lysosomal storage disorders or reducing seizures), is sufficient to achieve such therapeutic effect. In all embodiments described herein, preferably, the subject (or "patient") is human. The expression "mg total/day" in the context of doses is intended to refer to a daily dose of X mg. For example, a dose of 9 mg total/day of nizubaglustat means that 9 mg of nizubaglustat is administered to the patient in a given day. This can be given in a single dose or multiple (e.g. 2, 3 or 4) doses throughout the day. The expression "mg total/day" can equally be expressed as "mg/day" and have the same meaning.

The term "composition" as used herein is intended to encompass a product comprising the specified ingredients in the specified amounts, as well as any product which results, directly or indirectly, from combination of the specified ingredients in the specified amounts. Such term in relation to a pharmaceutical composition is intended to encompass a product comprising nizubaglustat and optionally additional ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition comprising nizubaglustat for use as described herein, and optionally a pharmaceutically acceptable carrier, diluent or excipient. By "pharmaceutically acceptable" it is meant the carrier, diluent or excipient (if present) must be compatible with the other ingredients of the formulation and not deleterious to the recipient thereof.

The term "nizubaglustat" is intended to encompass a potent dual inhibitor of glucosylceramide synthase (GCS; EC:2.4.1.80) and non-lysosomal glucosylceramidase (NLGase, GBA2, UniProt code: Q9HCG7; EC 3.2.1.45), which is currently undergoing Phase II study for treating GM2 gangliosidosis and Niemann-Pick disease type C. Nizubaglustat has formula (I), as shown below (PubChem CID: 101882900, CAS Number: 1633666-49-5).

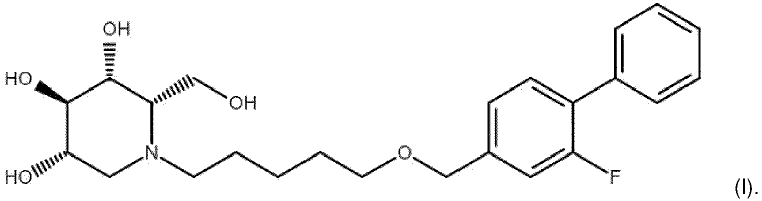

The invention provides nizubaglustat for use in treating a lysosomal storage disorder in a human subject suffering from the lysosomal storage disorder, wherein nizubaglustat is administered to the subject at a dose of between about 3mg - about 9mg total/day.

The lysosomal storage disorder may be GM2 gangliosidosis (e.g. Tay Sachs disease, Sandhoff disease or GM2 activator deficiency), Niemann-Pick disease type A, B, and C (transmembrane protein defect C1, soluble non-enzymatic protein defect C2), Gaucher disease types 1, 2, and 3, Pompe disease (glycogen storage disease type II), Fabry disease (classic and late-onset types), metachromatic leukodystrophy, Globoid leukodystrophy (Krabbe disease), and/or GM1 gangliosidosis types 1, 2, and 3. Preferably, the lysosomal storage disorder is GM2 gangliosidosis. Preferably, the lysosomal storage disorder is Niemann-Pick disease (e.g. type C).

In a healthy individual, the core glycosphingolipid, glucosylceramide, is hydrolysed in lysosomes by acid glucosylceramidase (also called glucocerebrosidase or GCAse, EC 3.2.1.45, UniProt code: P04062). In addition, the non-lysosomal glucosylceramidase (NLGase, GBA2, UniProt code: Q9HCG7) residing in the cytoplasm is also capable of processing glucosylceramide. As a result, both GCase and NLGase are involved in neuropathological effects observed is several lysosomal storage diseases (or disorders).

In patients with a lysosomal storage disorder, defects in glycosphingolipid biosynthesis or degradation occur, resulting in abnormal levels of glucosylceramide and/or other glycosphingolipids.

Thus, the lysosomal storage disorder may be associated with abnormal levels of cytosolic or lysosomal glucosylceramide and/or higher levels of glycosphingolipids. Nizubaglustat is a potent dual inhibitor of glucosylceramide synthase and non-lysosomal glucosylceramidase (NLGase, GBA2, UniProt code: Q9HCG7).

The dose of nizubaglustat administered to the subject may be between 3mg - 9mg total/day. The dose of nizubaglustat administered to the subject may be about 3mg total/day or about 9mg total/day. The dose of nizubaglustat administered to the subject may be 3mg total/day or 9mg total/day. Preferably, the dose of nizubaglustat administered to the subject is 9mg total/day.

The subject may be between 0-100, between 0-75, between 0-50, between 2-50, between 4-50 years of age. The subject may be above 12 years of age. The subject may be under 21 years of age. The subject may be under 19, or under 18 years of age. The subject may be between 0-21, between 0-19, between 0-18, between 1-19, between 1-18, between 12-21, between 12-19, or between 12-18 years of age. Preferably, the subject is under 21 years of age, for example, between 12-21 years of age. More preferably, the subject is under 19 years of age, for example, between 12-19 or between 12-18 years of age. The subject may be between 28 days and 30 years of age. The subject may be an infant (i.e. between 28 days and 12 months of age). The subject may be a toddler (i.e. between 12 months and 24 months of age). The subject may be a child (i.e. between 24 months and 12 years of age). The subject may be an adolescent (i.e. between 12 years and 17 years of age). The subject may be a young adult (i.e. between 17 years and 30 years of age). The invention is, however, also applied to adults above 30 years of age in some embodiments.

Nizubaglustat may be administered to the subject for at least 3 weeks, at least 4 weeks, at least 6 weeks, at least 8 weeks, at least 10 weeks, at least 3 months, at least 6 months, at least 1 year, at least 2 years, at least 3 years, at least 4 years, at least 5 years, or at least 10 years. Nizubaglustat may be administered to the subject throughout the life of the subject.

Nizubaglustat may be administered to the subject orally. Nizubaglustat may be administered to the subject daily, for example once daily or twice daily. Preferably, nizubaglustat is administered to the subject once daily. In some embodiments, nizubaglustat may be administered to the subject every 2, 3, 4, or 5 days.

It will be appreciated that the treatment of a lysosomal storage disorder may result in the elimination, alleviation or amelioration of one or more symptoms of a lysosomal storage disorder and need not necessarily eliminate, alleviate or ameliorate every symptom of a lysosomal storage disorder that the patient experienced prior to treatment.

By 'elimination' of a symptom is meant that the subject essentially no longer experiences that symptom. By 'alleviation' of a symptom is meant that the subject experiences a significant improvement in the symptom. The alleviation is preferably from severe to mild. By 'amelioration' of a symptom is meant that the subject experiences a modest improvement in the symptom.

Thus, in particular embodiments, the treatment, use and the like provided herein may result in the elimination, alleviation or amelioration of one or more symptoms of a lysosomal storage disorder. For example, one or more symptoms may be alleviated and one or more different symptoms may be ameliorated.

The symptoms of a lysosomal storage disorder (e.g. GM2 gangliosidosis) include at least one of muscle weakness, decreased movement, loss of motor skills, increased reaction to loud noises, seizures, vision loss, hearing loss, intellectual disability, an eye abnormality, enlarge organs, bone abnormalities, speech difficulties, loss of cognitive function, loss of muscle coordination, and/or psychiatric problems. Nizubaglustat may be used to alleviate, eliminate and/or ameliorate at least one symptom of a lysosomal storage disorder in a subject suffering from the lysosomal storage disorder. Preferably, nizubaglustat alleviates, eliminates and/or ameliorates at least 2, at least 3, at least 4, or at least 5 symptoms of a lysosomal storage disorder. For example, nizubaglustat may improve motor skills and muscle coordination in a subject.

Thus, alternatively viewed, provided is a method of eliminating, alleviating and/or ameliorating one or more of the above symptoms by administering a dose of between 3 mg - 9 mg total/day of nizubaglustat to a subject in need thereof.

The symptoms of a lysosomal storage disorder may be assessed in a subject using a Scale for the Assessment and Rating of Ataxia (SARA) score. The Scale for the Assessment and Rating of Ataxia (SARA) is a short, reliable and easy to use clinical score to assess the core symptoms of ataxia (Schmitz-Hubsch et al. "Scale for the assessment and rating of ataxia"; Neurobiology (2006), which is incorporated herein by reference). It consists of 8 items that sum up to a maximum of 40 points with higher scores indicating more severe ataxia. In more detail, the eight items (or domains) evaluated by SARA are gait (0-8 points), stance (0-6 points), sitting (0-4 points), speech disturbance (0-6 points), finger-chase test (0-4 points), nose-finger test (0-4 points), fast-alternating-movement test (0-4 points), and heel-shin slide (0-4 points). Scores of 0 indicate normal function; higher scores are indicative of increasing dysfunction. The SARA has been carefully validated in different kinds of ataxia and is widely used in movement disorder clinics, rehabilitation centers, and for clinical studies.

Administration of nizubaglustat at a dose of between 3 mg - 9 mg total/day may reduce a Scale for the Assessment and Rating of Ataxia (SARA) score in the subject as compared to the SARA score before administration of nizubaglustat. That is to say that the administration of nizubaglustat may reverse the progression of a disease (e.g. by reversing some of the symptoms of the disease).

Administration of nizubaglustat may eliminate, alleviate or ameliorate symptoms of a lysosomal storage disorder.

The SARA score may be reduced by at least 1, at least 1.1, at least 2, at least 3, at least 4, or at least 5. Preferably, the SARA score is reduced by at least 3, or at least 4.

The SARA score may be reduced to a certain level and then maintained at that level for a period of time (e.g. for 6 months, 12 months, 2 years, or 3 years) or for as long as the subject continues to undergo the nizubaglustat treatment. For example, the SARA score may be reduced from 33 to 29 and then maintained at that level for a period of time (or for as long as the subject undergoes the nizubaglustat treatment).

The reduction in the SARA score may take place upon continued (e.g. daily) administration of nizubaglustat for at least 2 weeks, at least 3 weeks, at least 4 weeks, at least 2 months, 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years. Preferably, the reduction in the SARA score takes place after at least 3, preferably at least 4 weeks, of continued (e.g. daily) administration of nizubaglustat.

Administration of nizubaglustat at a dose of between 3 mg - 9 mg total/day may stabilize the SARA score in the subject. That is to say that the SARA score and the associated disease symptoms might be maintained at the same level throughout the nizubaglustat treatment. Therefore, administration of nizubaglustat may result in stopping or slowing the progression of a disease (e.g. lysosomal storage disorder).

The SARA score may stabilize for at least 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years upon continued (e.g. daily) administration of nizubaglustat.

The subject may suffer from seizures. That is to say that in addition to other symptoms associated with a lysosomal storage disorder, the subject may also experience seizures. Seizures may be of any type, for example, general seizures bilateral seizures or focal onset seizures. Administration of nizubaglustat may reduce the duration and/or frequency of seizures in a human subject suffering from seizures.

Thus, the invention also provides nizubaglustat for use in reducing the duration and/or frequency of seizures in a human subject suffering from seizures, wherein nizubaglustat is administered to the subject at a dose of between about 3mg - about 9mg total/day. Preferably, the dose is 9 mg total/day.

Preferably, the subject suffers from a lysosomal storage disorder.

The dose of nizubaglustat may reduce the plasma levels of GlcCer C16 by at least 50% or at least 60% as compared to the levels before administration of nizubaglustat. Preferably, the dose of nizubaglustat reduces the plasma levels of GlcCer C16 by at least 75% as compared to the levels before administration of nizubaglustat. More preferably, the dose of nizubaglustat reduces the plasma levels of GlcCer C16 by at least 80% as compared to the levels before administration of nizubaglustat.

The levels of GlcCer C16 may be reduced after at least 1 day, at least 1 week, at least 2 weeks, at least 3 weeks, at least 4 weeks, at least 2 months, or at least 3 months of continued (e.g. daily) administration of nizubaglustat. Preferably, the levels of GlcCer C16 are reduced by at least 75% as compared to the levels before administration of nizubaglustat after continued (e.g. daily) administration of nizubaglustat for at least 4 weeks.

The dose of nizubaglustat may reduce the plasma levels of GlcCer C18 by at least 25% as compared to the levels before administration of nizubaglustat. Preferably, the dose of nizubaglustat reduces the plasma levels of GlcCer C18 by at least 30% as compared to the levels before administration of nizubaglustat. More preferably, the dose of nizubaglustat reduces the plasma levels of GlcCer C18 by at least 35% as compared to the levels before administration of nizubaglustat.

The levels of GlcCer C16 may be reduced after at least 1 day, at least 1 week, at least 2 weeks, at least 3 weeks, at least 4 weeks, at least 2 months, or at least 3 months of continued (e.g. daily) administration of nizubaglustat. Preferably, the levels of GlcCer C18 are reduced by at least 35% as compared to the levels before administration of nizubaglustat after continued (e.g. daily) administration of nizubaglustat for at least 4 weeks.

GlcCer C16 and C18 are useful markers for the determination of the activity of GCS. GCS is an enzyme responsible for the synthesis of glucosylceramide (GlcCer). The inhibitors of GCS reduce the levels of GlcCer and mitigate accumulation within lysosomes.

The levels of GlcCer C16 and C18 can be measured by methods known in the art. For example, blood samples can be collected before administration of nizubaglustat and at various time points (e.g. after 2 hours, 6 hours or 12 hours) after the administration of nizubaglustat. The concentrations of GlcCer C16 and C18 can be determined using validated high-performance liquid chromatography-tandem mass spectrometry (e.g. in multiple-reaction-monitoring model) (see e.g. Alhusban et al. "High Performance Liquid Chromatography-Tandem Mass Spectrometry Method for Correlating the Metabolic Changes of Lactate, Pyruvate and L-Glutamine with Induced Tamoxifen Resistant MCF-7 Cell Line Potential Molecular Changes"; Molecules (2021), incorporated herein by reference). Concentrations can then be calculated by interpolation from a calibration curve.

The dose of about 3mg - about 9mg total/day (e.g. 9 mg total/day) of nizubaglustat preferably does not trigger serious or severe treatment-emergent adverse reactions (or events) in the subject. For example, unlike miglustat, which is used to treat different lysosomal storage disorders, nizubaglustat at a dose of about 3mg - about 9mg total/day does not cause adverse gastrointestinal side effects, such as diarrhoea or vomiting. As shown in Example 3, nizubaglustat at a dose of about 3mg - about 9mg total/day is well tolerated by the subject. Thus, the dose of about 3mg - about 9mg total/day is safe to use in human subjects.

Serious or severe treatment-emergent adverse reactions (or effects) include, for example, nervous system disorders, reproductive system and breast disorders, or vascular and/or heart disorders (e.g. haematoma or heart attack). Nizubaglustat may occasionally trigger mild treatment emergent adverse effects, for example, skin disorders, such as dermatitis. However, these are easily managed with the help of topical ointments and the like.

Nizubaglustat may be administered to the subject in a crystalline form. The crystalline form may display a reflection, stated as a 2Θ value, at 17.2 ± 0.2°, 17.8 ± 0.2°, 21.2 ± 0.2° and/or 22.4 ± 0.2°, in an X-ray powder diffraction pattern. The crystalline form may display a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern. The crystalline form may further display one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 19.3 ± 0.2°, 22.9 ± 0.2° and 23.3 ± 0.2°, in an X-ray powder diffraction pattern.

Suitable crystalline forms of nizubaglustat are described in WO2022/069709, which is incorporated herein by reference.

Nizubaglustat may be in any crystal state. Nizubaglustat may be in a crystalline salt or a crystalline free base (non-ionized) form. Preferably, nizubaglustat is in a crystalline free base form.

The term "strongest reflection" describes the highest peak of an X-ray powder diffraction pattern. The height of a peak of a powder X-ray diffraction pattern is determined based on the X-ray intensity (in counts or counts/sec units). Thus, the strongest reflection is a reflection that shows the highest X-ray intensity in the X-ray powder diffraction pattern.

Unless explicitly stated to the contrary, all X-ray powder diffraction patterns are determined using copper K-alpha radiation at about 25 °C.

The crystalline forms of a compound can be characterized by a differential scanning calorimetry (DSC) thermogram. Nizubaglustat may be characterized by a DSC thermograph, which shows an onset of endothermic heat flow at about 87 °C and/or a melting point of about 92.4°C. Accordingly, nizubaglustat may have a melting point of 89 °C to 96 °C, as determined by DSC. Preferably, the melting point is between 90 °C to 95 °C, as determined by DSC. More preferably still, the melting point is between 91 °C to 94 °C, as determined by DSC. A relatively high melting point (typically greater than about 80°C) of a therapeutic compound favours resistance to decomposition thereby facilitating the storage and increasing shelf life of the therapeutic compound, which is desirable for any pharmaceutical agent.

The crystalline forms of a compound can be characterized by their hygroscopicity. Hygroscopicity of a product expresses the increase or decrease in its water content as a function of relative humidity at a certain temperature. Substantially non-hygroscopic products exhibit no or only a slight change in their water content as a consequence of variations in relative humidity. In strongly hygroscopic products, water content may vary widely. Accordingly, nizubaglustat may be substantially non-hygroscopic. When preparing pharmaceutical compositions and formulations for use in such tablets, it is highly desirable to have a crystalline form of the therapeutic compound having low levels of hygroscopicity and/or low levels of deliquescence thereby allowing the compound to be compressed into a desired shape or size.

Substantially non-hygroscopic substances show a water absorption of less than about 2% at a relative humidity of about 95% measured at a temperature of about 25°C. Preferably, water absorption is less than about 1 % at a relative humidity of about 95% measured at a temperature of about 25°C. The values of water absorption are obtained by measuring the mass gain of the tested crystalline form at a relative humidity of about 95% and a temperature of about 25°C relative to the initial mass.

The crystalline form of nizubaglustat may absorb 0% to 2% water at a relative humidity of about 95% at a temperature of about 25°C. The crystalline form of nizubaglustat may absorb 0% to 1.5% water at a relative humidity of about 95% at a temperature of about 25°C. The crystalline form of nizubaglustat may absorb 0% to 1% water at a relative humidity of about 95% at a temperature of about 25°C.

Nizubaglustat may have an alternative (less preferred) crystalline form, in which the crystalline form displays a reflection, stated as a 2Θ value, at 16.9 ± 0.2°, in an X-ray powder diffraction pattern, wherein the reflection at 16.9 ± 0.2° is one of the four strongest reflections in the X-ray powder diffraction pattern. The crystalline form of nizubaglustat may further display one or more reflections, stated as a 2Θ value, at one or more of 15.2 ± 0.2°, 16.1 ± 0.2°, 16.5 ± 0.2°, 18.9 ± 0.2°, 23.1 ± 0.2°, 25.5 ± 0.2°, 27.7 ± 0.2° and 28.5 ± 0.2°, in an X-ray powder diffraction pattern.

The crystalline form of nizubaglustat may be characterized by a good solubility in water. Accordingly, nizubaglustat may have water solubility of about 75 mg/mL to about 85 mg/mL, measured at about 25°C. Methods for measuring solubility are known in the art; for example, shake-flask method, sonication, column elution method and ultraviolet or visible spectroscopy method. Unless explicitly stated to the contrary, water solubility is determined using the shake-flask method and/or sonication.

In the uses and methods described herein, nizubaglustat may be administered to the subject as a pharmaceutical composition. Thus, the invention also provides a pharmaceutical composition comprising nizubaglustat, as described herein, at a dose of between about 3mg - about 9mg and, optionally, at least one pharmaceutically acceptable carrier. Preferably, nizubaglustat is at the dose of 9mg total/day.

The pharmaceutical composition may have a pH value of between 2.0 and 8.0, between 3.0 and 7.0, between 4.0 and 7.5, between 4.0 and 6.0, or between 4.5 and 5.5. Preferably, the pharmaceutical composition has a pH value of between 4.5 and 5.5. The pharmaceutical composition may further comprise an acid and/or a base. For example, the pharmaceutical composition may comprise an acid to lower the pH to a desired pH value. The base may be used to increase the pH to a desired pH value. The skilled person would easily be able to determine suitable acids and bases to manipulate the pH value of the pharmaceutical composition. For example, the acid may be citric acid, acetic acid, or hydrochloric acid. For example, the base may be sodium hydroxide or potassium hydroxide.

The pharmaceutical compositions of the invention are typically prepared by pharmaceutically acceptable carrier and one or more optional ingredients. If necessary or desired, the resulting uniformly blended mixture can then be shaped or loaded into tablets, capsules, pills, canisters, cartridges, dispensers, and the like using conventional procedures and equipment.

When intended for oral administration in a solid dosage form (i.e., as capsules, tablets, pills and the like), the pharmaceutical compositions of the invention will typically comprise nizubaglustat as the active ingredient. The pharmaceutical composition of the invention may comprise nizubaglustat and no other ingredient. The pharmaceutical composition of the invention may be contained in a capsule. The pharmaceutical composition of the invention may be contained in the capsule without any other ingredient. The capsule may be a gelatine capsule or a hydroxypropyl methylcellulose (HPMC) capsule. Alternatively, the pharmaceutical composition of the invention may comprise nizubaglustat as the active ingredient and one or more pharmaceutically acceptable carriers. Suitable pharmaceutically acceptable carriers would be known by the person skilled in the art, for example, fats, water, physiological saline, alcohol (e.g., ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose, agents regulating the pH of the composition, such as bases and/or acids; and other conventionally used additives such as gelatin, talc, plant oil and gum arabic.

The oral dosage form may further be a capsule delayed release, in which nizubaglustat is enclosed within either a hard or soft soluble container made from a suitable form of gelatin or hydroxypropyl methylcellulose (HPMC), and which releases nizubaglustat at a time other than promptly after administration, whereby enteric-coated articles are delayed release dosage forms. Capsule delayed release pellets, in which nizubaglustat is enclosed within either a hard or soft container or "shell" are also useful. In these cases, nizubaglustat itself is in the form of granules to which enteric coating has been applied, thus delaying release of the agent until its passing into the intestine. Capsule extended release and capsule film-coated extended release are also useful.

Additionally, the capsule may be covered in a designated film coating which releases nizubaglustat in such a manner to allow at least a reduction in dosing frequency as compared to that presented as a conventional dosage form.

In some embodiments, nizubaglustat may be dissolved or suspended in a liquid vehicle or formulated as a granule (a small particle or grain), a pellet (a small sterile solid mass consisting of a highly purified nizubaglustat), with or without excipients, made by the formation of granules, or by compression and moulding), or a pellet coated extended release.

Other forms include pills (a small, round solid dosage form containing nizubaglustat) intended for oral administration), powder (an intimate mixture of dry, finely divided nizubaglustat with one or more pharmaceutically acceptable additives that may be intended for internal or external use), elixir (a clear, pleasantly flavoured, sweetened hydroalcoholic liquid containing dissolved nizubaglustat; it is intended for oral use), chewing gum (a sweetened and flavoured insoluble plastic material of various shapes which when chewed, releases nizubaglustat into the oral cavity), syrup (an oral solution containing nizubaglustat and high concentrations of sucrose or other sugars; the term has also been used to include any other liquid dosage form prepared in a sweet and viscid vehicle, including oral suspensions), tablet (a solid dosage form containing nizubaglustat with or without suitable diluents), tablet chewable (a solid dosage form containing nizubaglustat with or without suitable diluents that is intended to be chewed, producing a pleasant tasting residue in the oral cavity that is easily swallowed and does not leave a bitter or unpleasant after-taste), tablet coated or tablet delayed release, dispersible tablet, tablet effervescent, tablet extended release, tablet film coated, or tablet film coated extended release where the tablet is formulated in such manner as to make the contained nizubaglustat available over an extended period of time following ingestion.

In other forms, a tablet for solution, tablet for suspension, tablet multilayer, tablet multilayer extended release may be provided, where the tablet is formulated in such manner as to allow at least a reduction in dosing frequency as compared to that presented as a conventional dosage form. A tablet orally disintegrating, tablet orally disintegrating delayed release, tablet soluble, tablet sugar coated, osmotic, and the like are also suitable.

As used herein, injectable and infusion dosage forms include, but are not limited to, a liposomal injectable, which either consists of or forms liposomes (a lipid bilayer vesicle usually composed of phospholipids which is used to encapsulate nizubaglustat); an injection, which includes a sterile preparation intended for parenteral use; an emulsion injection, which includes an emulsion consisting of a sterile, pyrogen-free preparation intended to be administered parenterally; or a lipid complex injection.

Other forms include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate nizubaglustat, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

As used herein, a "solution injection" refers to a liquid preparation containing nizubaglustat dissolved in a suitable solvent or mixture of mutually miscible solvents that is suitable for injection. A "solution concentrate injection" refers to a sterile preparation for parenteral use which, upon the addition of suitable solvents, yields a solution conforming in all respects to the requirements for injections.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate nizubaglustat, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of nizubaglustat which is preferably isotonic with the blood of the recipient, but this is not essential.

As used herein, inhalation dosage forms include, but are not limited to, an aerosol (a product that is packaged under pressure and contains nizubaglustat which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing nizubaglustat, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains nizubaglustat, in the form of a powder, that is released upon activation of an appropriate valve system. An aerosol spray is an aerosol product which utilizes a compressed gas as the propellant to provide the force necessary to expel the product as a wet spray and being applicable to solutions of nizubaglustat in aqueous solvent(s).

The pharmaceutical composition comprising nizubaglustat may also be administered transdermally or transmucosally using known delivery systems and excipients. For example, the pharmaceutical composition can be admixed with permeation enhancers such as propylene glycol, polyethylene glycol monolaurate, azacycloalkan-2-ones and the like, and incorporated into a patch or similar delivery system. Additional excipients including gelling agents, emulsifiers, and buffers, may also be used. As used herein, transdermal dosage form includes, but is not limited to, a patch (a drug delivery system that often contains an adhesive backing that is usually applied to an external site on the body, whereby the ingredients (including nizubaglustat) either passively diffuse from, or are actively transported from, some portion of the patch, and whereby depending upon the patch, the ingredients (including nizubaglustat) are either delivered to the outer surface of the body or into the body. Various types of transdermal patches such as matrix, reservoir and others are known in the art.

As used herein, the topical dosage form includes various dosage forms known in the art such as lotions (an emulsion, liquid dosage form, whereby this dosage form is generally for external application to the skin), lotion augmented (a lotion dosage form that enhances delivery of nizubaglustat, whereby augmentation does not refer to the strength of nizubaglustat in the dosage form), gels (a semisolid dosage form that contains a gelling agent to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances compound delivery, whereby augmentation does not refer to the strength of nizubaglustat in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances compound delivery, whereby augmentation does not refer to the strength of nizubaglustat in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains nizubaglustat and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

Preferably the pharmaceutical composition comprises one or more further pharmaceutically active agents (e.g. anti-inflammatory agents, such as Aspirin). This combination therapy involves using nizubaglustat combined with one or more of the further pharmaceutically active agents, either formulated together (for example, packaged together in a single formulation) or formulated separately (for example, packaged as separate unit dosage forms).

The methods and uses described herein may be *in vitro* methods (or uses) or *in vivo* methods (or uses).

The invention also provides a method of treating a lysosomal storage disorder in a human subject suffering from the lysosomal storage disorder, the method comprises administering to the subject nizubaglustat at a dose of between about 3mg - about 9mg total/day.

The invention also provides a method of reducing the frequency and duration of seizures in a human subject suffering from seizures, the method comprises administering to the subject nizubaglustat at a dose of between about 3mg - about 9mg total/day.

The features discussed in the context of uses and pharmaceutical compositions described herein apply *mutatis mutandis* in the context of methods described herein.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The invention is further disclosed in the following clauses:
1. Nizubaglustat for use in treating a lysosomal storage disorder in a human subject suffering from the lysosomal storage disorder, wherein nizubaglustat is administered to the subject at a dose of between 3mg - 9mg total/day.
2. Nizubaglustat for use of clause 1, wherein the subject is under 21 years of age, preferably under 18 years of age.
3. Nizubaglustat for use of clause 1 or clause 2, wherein the subject is between 12 and 18 years of age.
4. Nizubaglustat for use of any one of clauses 1-3, wherein the dose is about 9 mg total/day.
5. Nizubaglustat for use of any one of clauses 1-4, wherein the dose reduces a Scale for the Assessment and Rating of Ataxia (SARA) score in the subject as compared to the SARA score before administration of nizubaglustat.
6. Nizubaglustat for use of clause 5, wherein the dose reduces the SARA score by at least 1.1, preferably by at least 3, or at least 4.
7. Nizubaglustat for use of clause 5 or clause 6, wherein the dose reduces the SARA score in the subject upon continued (e.g. daily) administration for at least 2 weeks, at least 3 weeks, at least 4 weeks, at least 2 months, 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years.
8. Nizubaglustat for use of any one of clauses 1-7, wherein the dose stabilises the SARA score for at least 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years upon continued (e.g. daily) administration.
9. Nizubaglustat for use of any one of clauses 1-8, wherein the subject suffers from seizures.
10. Nizubaglustat for use of any one of clauses 1-9, wherein the dose reduces the frequency and/or duration of seizures in the subject.
11. Nizubaglustat for use of any one of clauses 1-10, wherein nizubaglustat is administered to the subject for at least 3 weeks, preferably for at least 4 weeks.
12. Nizubaglustat for use of any one of clauses 1-11, wherein nizubaglustat is administered orally.
13. Nizubaglustat for use of any one of clauses 1-12, wherein nizubaglustat is administered daily or every 2 days, preferably once daily.
14. Nizubaglustat for use of any one of clauses 1-13, wherein the dose reduces the plasma GlcCer C16 levels by at least 60%, preferably at least 75%, more preferably by at least 80% as compared to the levels before administration of nizubaglustat.
15. Nizubaglustat for use of any one of clauses 1-14, wherein the dose reduces the plasma GlcCer C18 levels by at least 25% as compared to the levels before administration of nizubaglustat, preferably at least 30%, more preferably by at least 35%, on daily administration for at least 4 weeks.
16. Nizubaglustat for use of any one of clauses 1-15, wherein the dose reduces the plasma GlcCer C16 levels by at least 50%, as compared to the levels before administration of nizubaglustat, preferably at least 60%, more preferably by at least 70%, on daily administration for at least 4 weeks.
17. Nizubaglustat for use of any one of clauses 1-16, wherein the dose does not trigger serious or severe treatment-emergent adverse reactions in the subject.
18. Nizubaglustat for use of any one of clauses 1-17, wherein the lysosomal storage disease is GM2 gangliosidosis (e.g. Sandhoff disease) and/or Niemann-Pick disease (e.g. Niemann-Pick disease type C).
19. Nizubaglustat for use of any one of clauses 1-18, wherein the blood concentration of nizubaglustat is between 12-400 ng/mL after administration, preferably between 25-240 ng/mL after administration.
20. Nizubaglustat for use of any one of clauses 1-19, wherein nizubaglustat is in a crystalline form.
21. Nizubaglustat for use of clause 20, wherein the crystalline form displays a reflection, stated as a 2Θ value, at 17.2 ± 0.2°, 17.8 ± 0.2°, 21.2 ± 0.2° and/or 22.4 ± 0.2°, in an X-ray powder diffraction pattern.
22. Nizubaglustat for use of clause 20 or clause 21, wherein the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.
23. Nizubaglustat for use of any one of clauses 20-22, further displaying one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 19.3 ± 0.2°, 22.9 ± 0.2° and 23.3 ± 0.2°, in an X-ray powder diffraction pattern.
24. A pharmaceutical composition comprising nizubaglustat at a dose of between 3mg - 9mg and at least one pharmaceutically acceptable carrier.
25. The pharmaceutical composition of clause 24, wherein nizubaglustat is at a dose of 9 mg total/day.

### EXAMPLES

The following non-limiting examples further illustrate the present invention.

### Example 1 - Dose determination for nizubaglustat based on clinical data

Nizubaglustat has been administered in humans aged 12 years and older with single doses of 3 and 9 mg and multiple doses of 3 and 9 mg. In a Phase 2 study, adolescents (12-17) and adults (>18) with GM2 gangliosidosis or NPC disease received nizubaglustat 3 mg or 9 mg daily over 12 weeks (and beyond - 52 weeks extension). The study demonstrated a dose- and exposure- dependent effect on the PD biomarker glucosylceramide (GlcCer) C16. Preliminary PKPD modelling indicated a strong correlation between exposure and response using an indirect response model. More details are provided with reference to the results in the Figures below.

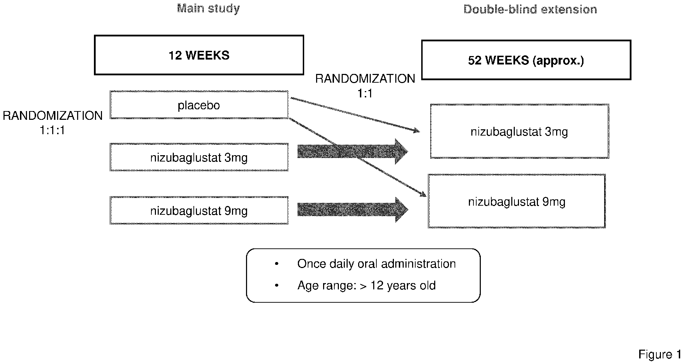

Figure 1 shows the phase 2 study protocol for using nizubaglustat to treat lysosomal storage disorders, such as GM2 gangliosidosis (GM2) or Niemann-Pick disease type C (NPC). Doses of placebo, 3 and 9 mg and multiple doses of 3 and 9 mg were administered to human patients suffering from lysosomal storage disorders, such as GM2 gangliosidosis (GM2) or Niemann-Pick disease type C (NPC). The doses were administered daily via oral administration. The study was carried out for at least 52 weeks to assess safety profile and effective dose. The study consisted of a 3-month placebo-controlled part which was used to determine PK and PD parameters as well as monitor safety of the subjects and tolerability of nizubaglustat. These parameters were then utilized to determine the optimal dose for the phase 3 program in conjunction with preclinical data and modelling.

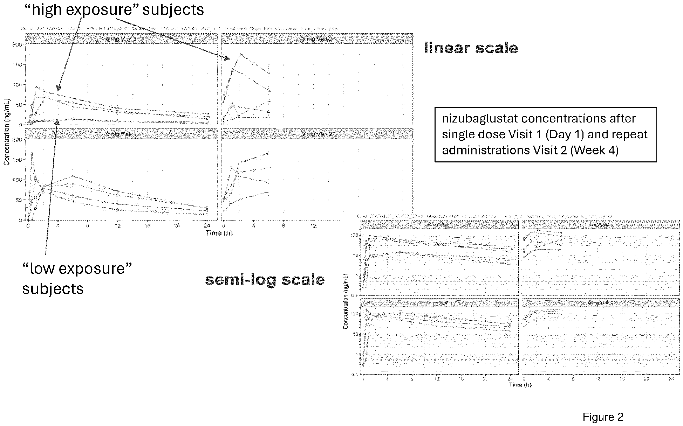

Figure 2 illustrates the pharmacokinetic data, which show measurable concentrations of nizubaglustat across all patients dosed and low variability in the 9mg group. In the figure, the nizubaglustat concentrations were measured after single dose (day 1 of administration - Visit 1) and after repeat administration for 4 weeks (Visit 2). In 3 mg dose, a proportion of patients was categorized as "high exposure" subjects because they showed a higher concentration of nizubaglustat in their system, and as "low exposure" subjects because they showed a lower concentration of nizubaglustat in their system. No significant difference was observed between the two categories of patients for dose 3 mg in relation to the safety profile of nizubaglustat, or its effectiveness.

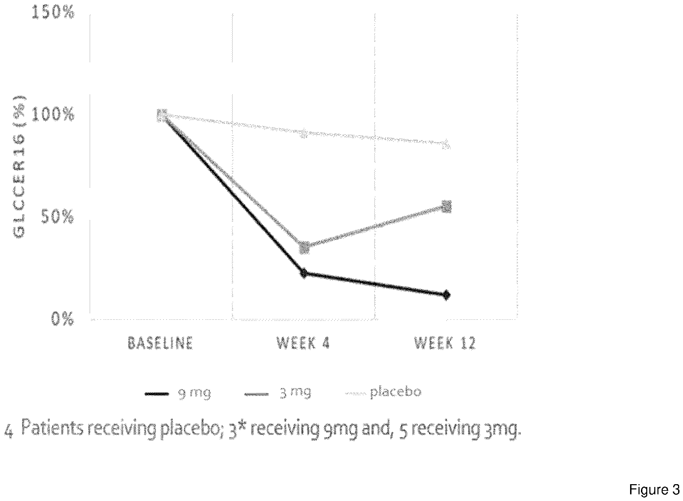

Figure 3 illustrates the glucosyl (beta) ceramide (GlcCer) C16 plasma level reductions across NPC and GM2 patients at the 3 mg and 9 mg doses (and placebo) over the period of 12 weeks of administration of nizubaglustat (or placebo). The reduction was greater (max 87% in week 12, and about 75% in week 4) for patients receiving 9 mg dose as compared to the patients receiving 3 mg dose (max 62% in week 4). No significant difference was observed for placebo patients. The baseline value indicates levels of GlcCer C16 from before administration of nizubaglustat.

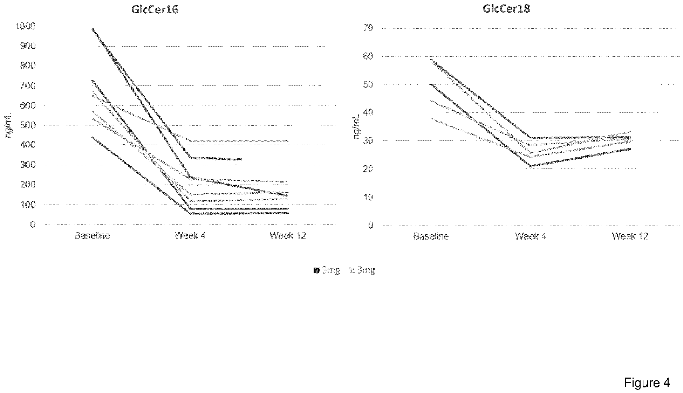

Figure 4 illustrates the summary of GlcCer C16 and GlcCer C18 plasma levels in the GM2 and NPC patients tested at 4 weeks and 12 weeks post-nizubaglustat administration. The data shows results for doses 9 mg and 3 mg. The data for individual patient is shown in Table 1 below:

**Table 1. GlcCer C16 and GlcCer C18 plasma levels in patients receiving nizubaglustat before administration (baseline) and at week 4 and week 8 or 12 of daily administration.**

| | | **GlcCer C16** | | | | **GlcCer C18** | | | |
|---|---|---|---|---|---|---|---|---|---|
| **Dose (mg)** | **Patient ID** | **Base -line** | **Week 4** | **Week 8** | **Week 12** | **Base-line** | **Week 4** | **Week 8** | **Week 12** |
| **3** | xxx03_ NPC | 646 | 420 | NA | 420 | 104 | 78.8 | NA | 84.7 |
| | xxx13_ NPC | 531 | 226 | NA | 215 | 65.3 | 42.4 | NA | 43.4 |
| | xxx04_ GM2 | 569 | 148 | NA | 161 | 44.1 | 28.3 | NA | 30.7 |
| | xxx05_ GM2 | 477 | 160 | NA | 135 | 58.4 | 25.6 | NA | 33.2 |
| | xxx02_ GM2 | 668 | 116 | NA | 127 | 37.8 | 24.2 | NA | 29.7 |
| **9** | xxx01_ NPC | 988 | 235 | NA | 142 | 68.1 | 41.5 | NA | 35.5 |
| | xxx12_ GM2 | 724 | 79.3 | NA | 76.4 | 58.7 | 30.9 | NA | 31.3 |
| | xxx15_ GM2 | 439 | 54.5 | NA | 57.6 | 50.0 | 21.0 | NA | 27.1 |
| | xxx23_ NPC | 984 | 337 | 316 | NA | 111 | 71.5 | 79.5 | NA |

The data show an average decrease in GlcCer C16 levels of 63% and 80% at week 4 for 3mg and 9mg doses, respectively. The data show an average decrease in GlcCer C18 levels of 37% and 40% at week 4 for 3mg and 9mg doses, respectively. The decreased level of GlcCer C16 remains stable in week 12 after daily administration of the drug for both doses.

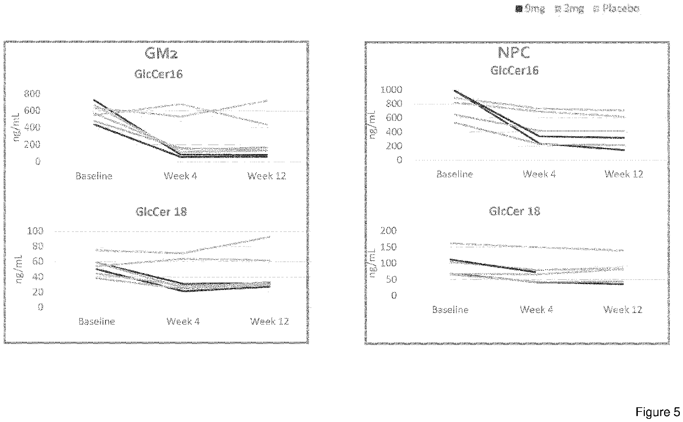

Figure 5 shows data as in Figure 4, with the split for GM2 and NPC patients and including placebo. The pharmacodynamic data showed a consistent, dose-dependent reduction of Glucosylceramides 16 and 18, which are targets of nizubaglustat.

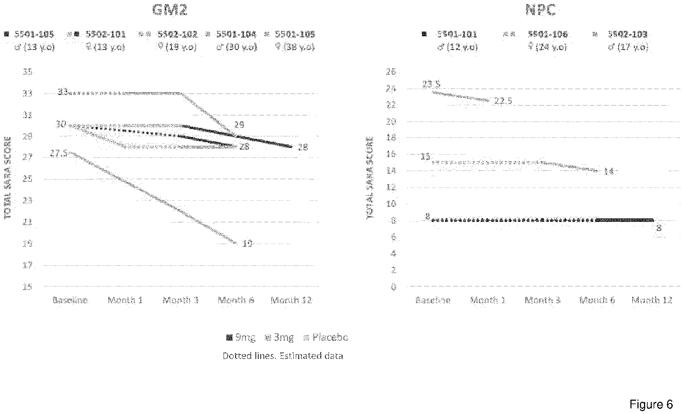

Figure 6 illustrates the Scale for the Assessment and Rating of Ataxia (SARA) data for phase 2 study. The Scale for the Assessment and Rating of Ataxia (SARA) is a short and easy to use clinical score to assess the core symptoms of ataxia. It consists of 8 items (gait, stance, sitting, speech disturbance, finger-chase, nose-finger test, fast alternating hand movements and heel-shin slide) that sum up to a maximum of 40 points with higher scores indicating more severe ataxia. The SARA has been carefully validated in different kinds of ataxia and is widely used in movement disorder clinics, rehabilitation centers, and for clinical studies (Full Ref). As shown in Figure 6, all patients for whom SARA data are available show an improvement in their scores in the study. Particularly the decreases in score by 4 and 8.5 points are clearly remarkable, given that a change of the SARA score by 1.1 at group level is seen as a clinically significant change. A lower score denotes a lower disease burden and fewer symptoms. In the context of progressive and/or degenerative diseases, such as GM2 gangliosidosis and NPC, the maintenance of the score at the same level for a period of time (e.g. 12 months) is also seen as a positive effect. These data support that nizubaglustat is an effective drug in treating these diseases in humans.

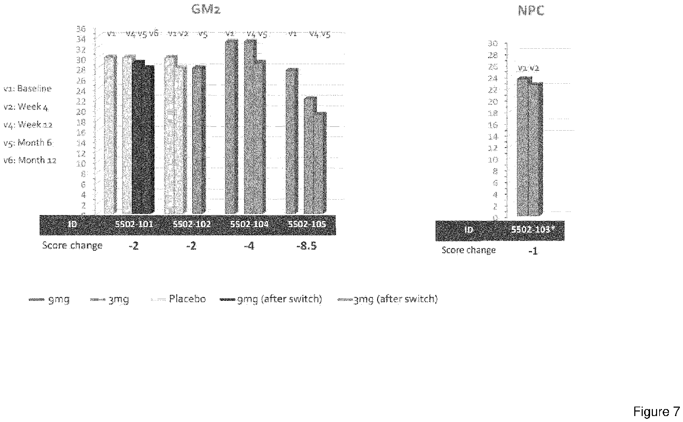

Figure 7 shows the SARA data at different time points in the study. The negative score change indicates an improvement of symptoms and therefore a positive therapeutic effect. The positive impact of nizubaglustat on SARA scores can be seen for all patients tested as early as 4 weeks in the study. This is unexpected as usually disease stabilization is the target for lysosomal storage disorders treatments, and the required longer administration of the active to achieve any positive effect is one of the reasons for the long duration of clinical trials (e.g. Mistry et al. (2015) "Effect of Oral Eliglustat on Splenomegaly in Patients With Gaucher Disease Type 1: the ENGAGE randomized clinical trial". JAMA: 313(17), or Patterson et al (2010) "Long-term miglustat therapy in children with Niemann-Pick disease type C". J Child Neurol : 25(3)). The "after swich" label indicates that the patient was initially on the placebo treatment and was switched to either 3mg or 9mg dose after 12 weeks in the study.

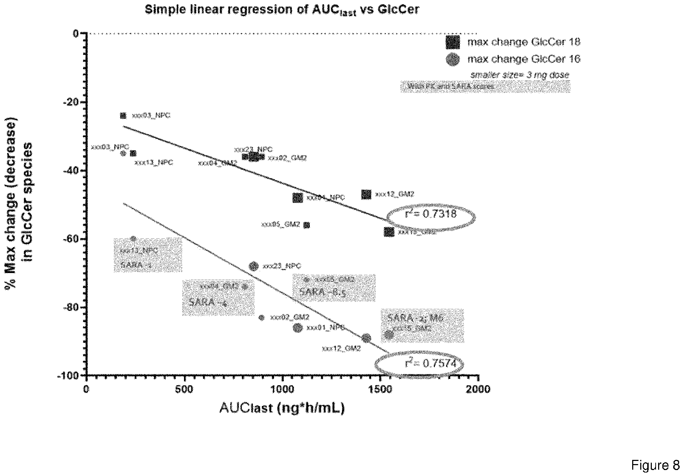

Figure 8 shows a plot of the correlation between the maximal % reduction in the GlcCer (at any time point) vs. the exposure (AUC) following the first visit (visit 1). During the first visit, the acquired blood samples cover sampling time-points that adequately reflect the 24-h period post dose, thus providing a very good measure of exposure of nizubaglustat.

While dose relies on an absorption phase, exposure is a more robust measurement of nizubaglustat levels that occurs in each patient. In this plot we first can see that increases in nizubaglustat exposure is strongly correlated with a decrease in GlcCer 16 (same is true for GlcCer 18, c.f. Table 2, below). This is expected as the drug inhibits the formation of GlcCer species via the inhibition of GCS (glucosylceramide synthase). Second, superimposed on this graph are the available SARA scores in which we observe an improvement or stabilization in SARA scores.

**Table 2. Correlation of the nizubaglustat exposure (AUCₗₐₛₜ) with the levels of plasma GleCer C16 and GleCer C18.**

| **Dose (mg)** | **Patient ID** | **Cₘₐₓ** | **AUCₗₐₛₜ** | **Disease type** | **max % change GlcCer 16** | **max % change GlcCer 18** |
|---|---|---|---|---|---|---|
| **3** | xxx03_NPC | 13.9 | 187 | NPC | -35% | -24% |
| | xxx13_NPC | 14.8 | 238 | NPC | -60% | -35% |
| | xxx04_GM2 | 68.6 | 809 | GM2 | -74% | -36% |
| | xxx05_GM2 | 93.6 | 1123 | GM2 | -72% | -56% |
| | xxx02_GM2 | 67.5 | 893 | GM2 | -83% | -36% |
| | | | | | | |
| **9** | xxx01_NPC | 99.3 | 1078 | NPC | -86% | -48% |
| | xxx12_GM2 | 89.8 | 1428 | GM2 | -89% | -47% |
| | xxx15_GM2 | 109 | 1544 | GM2 | -88% | -58% |
| | xxx23_NPC | 164 | 853 | NPC | -68% | -36% |

Both 3 and 9mg doses of nizubaglustat are capable of significantly reducing plasma GlcCer concentrations. Both doses are also associated with an improvement in the SARA score (i.e. reduction of the score). The nizubaglustat exposure drives the reduction of plasma GlcCer concentrations and the level of improvement of the SARA score.

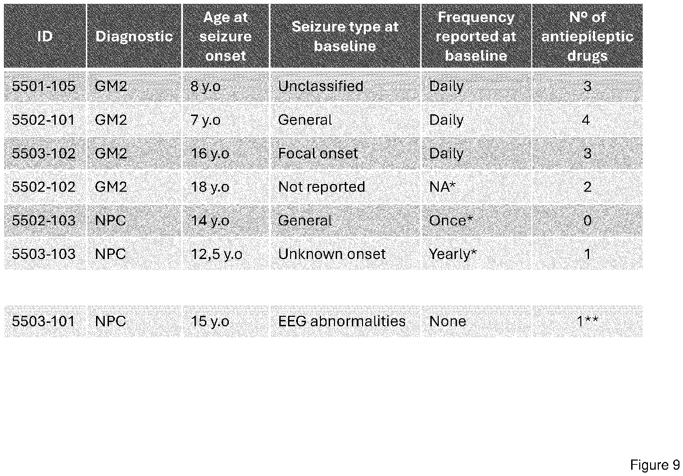

Figure 9 illustrates the type and frequency of seizures experienced by some of the patients before enrolling into the phase 2 study.

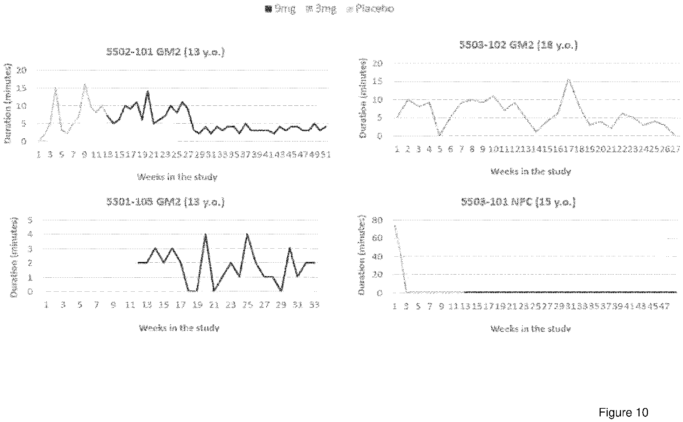

Figure 10 illustrates the duration of seizures experienced by the patients taking part in the phase 2 study. Nizubaglustat decreases the duration per week of seizures experienced by the patients taking part in the phase 2 study. For example, the duration of seizures in GM2 patient 5502-101 decreased from about 16 minutes in week 9 (when the patient received placebo) to about 4 minutes in each of weeks 29-51 of the study (when the patient received 9 mg nizubaglustat). The frequency of seizures has also decreased for the patients taking part in the phase 2 study and receiving either a 3mg or 9mg dose of nizubaglustat (data not shown).

### Example 2 - Pharmacokinetics analysis in patients suffering from NPC or GM2

Non-compartmental analysis of the Phase 2 Study involving patients with GM2 gangliosidosis or Niemann-Pick type C disease were conducted. The study analyzed the pharmacokinetics following the oral intake of nizubaglustat.

On the first day, maximum plasma concentrations were achieved approximately 2.25 hours after administering a 3mg dose and 3.5 hours after a 9mg dose. By the fourth week, the peak concentrations were reached around 2 hours post-dose for 3mg and 6 hours for 9mg of nizubaglustat, administered daily.

For the 3mg dose groups the maximum plasma exposure (Cₘₐₓ) geometric mean (CV%) values on Day 1 and Day 29 (week 4) were 38.9 ng/mL (69.0) and 66.5 ng/mL (74.8), respectively. The corresponding values for AUC₀₋₆ were 172 h.ng/mL (70.1) and 315 h.ng/mL (77.3), respectively. For the 9mg dose groups the maximum plasma exposure (Cₘₐₓ) mean values on Day 1 and Day 29 (week 4) were 112 ng/mL (28.8) and 119 ng/mL (32.2), respectively. The corresponding geometric mean values for AUC₀₋₆ were 433 h.ng/mL (3.38) on Day 1 and 565 h ng/mL (32.7) on Day 29, respectively.

Lambda-z and associated PK parameters could not be estimated. Following repeated once daily doses, nizubaglustat (AZ-3102) accumulated in plasma approximately 1.31-fold for the 3mg dose and 1.83 - fold for the 9mg dose group.

**Table 1. Geometric Mean (Geometric CV%) Plasma Pharmacokinetic Parameters for Nizubaglustat (AZ-3102)**

| **Dose** | **3mg** | **9mg** |
|---|---|---|
| ***Day 1*** | | |
| Number of Subjects | 5 | 4 |
| Tₘₐₓ^{a} (h) | 2.25 (1.10-6.17) | 3.50 (0.50-6.00) |
| Cₘₐₓ (ng/mL) | 38.9 (69.0) | 112 (28.8) |
| AUCₗₐₛₜ (ng.h/mL) | 515 (64.0) | 1193 (25.9) |
| AUC₀₋₆ (ng.h/mL) | 172 (70.1) | 433 (3.38) |

| ***Day 29*** | | |
|---|---|---|
| Number of Subjects | 5 | 4 |
| Tₘₐₓ^{a} (h) | 2.00 (1.10-6.08) | 6.00 (1.00-6.00) |
| Cₘₐₓ (ng/mL) | 66.5 (74.8) | 119 (32.2) |
| AUC₀₋₆ (ng.h/mL) | 315 (77.3) | 565 (32.7) |
| R_{Cmax} | 1.71 (47.6) | 1.06 (43.2) |
| R_{AUCtau} | 1.83 (49.3) | 1.31 (31.2) |

Following Oral Administration of a 3 and 9mg dose of Nizubaglustat (AZ-3102) once daily to GM2 or NPC patients - Day 1 and 29 Tₘₐₓ:time to maximal concentration (Cₘₐₓ); Cmax: maximal concentration; AUCₗₐₛₜ: AUC from time 0 to the last measurable concentration.; AUC₀₋₆:Area under the curve from the time 0 until the sampled measurement taken at 6 h. R_{Cmax}: accumulation ratio for Cmax from Day 1 to Day 29; R_{AUCtau}: accumulation ratio for AUCtau, where tau represents the dosing interval.

The PK parameters for nizubaglustat were derived by non-compartmental analysis of the concentration-time profiles in order to determine the maximum concentration (Cmax), time to maximum concentration (Tmax), the terminal elimination half-life (t1/2), and the area under the curve (AUC) from 0 to time of the last measured concentration above the limit of quantification (here 6hrs - AUC₀₋₆).

Overall, the pharmacokinetic in patients based on data from phase 2 study revealed a high variability especially in the 3mg dose groups. Three subjects had unusually high exposures (Cₘₐₓ and AUCₗₐₛₜ) which could be due to drug-drug interactions with other co-medications. In addition, the remaining two subjects in the 3mg dose groups displayed lower Cₘₐₓ and AUCₗₐₛₜ than observed in healthy volunteers. The 9mg dose group had similar exposures when comparing it to the results in healthy volunteers (phase 1 study: Luzy, Cecile Paquet, et al. "First-in-human single-dose study of nizubaglustat, a dual inhibitor of ceramide glucosyltransferase and non-lysosomal glucosylceramidase: Safety, tolerability, pharmacokinetics, and pharmacodynamics of single ascending and multiple doses in healthy adults." Molecular Genetics and Metabolism 141.1 (2024): 108113)).

### Example 3- Safety

Safety assessments consisted of monitoring and recording adverse events (AEs), measurement of protocol-specified safety laboratory assessments, vital signs, and electrocardiograms (ECGs). As seizures are common occurrences in NPC and GM2 patients, an emphasis was placed on recording baseline seizure status. As noted above, seizure diaries were implemented in the protocol and informed consent to capture changes in frequency and/or type of seizures that occurred during the study.

Overall, 6 (66.7%) participants who received nizubaglustat experienced at least 1 treatment emergent adverse event (TEAE), including 4 (100.0%) in the 9mg group and 2 (40.0%) in the 3mg group (Table 4). All 4 (100%) participants in the placebo group experienced at least 1 TEAE. Among participants who received nizubaglustat, 4 (44.4%) experienced a least 1 TEAE the Investigator considered related to the study drug, including 2 (50.0%) in the 9mg group and 2 (40.0%) in the 3mg group. In the placebo group, 2 (50.0%) participants experienced a TEAE the Investigator considered related to the study drug.

No participant experienced a TEAE that led to study drug interruption or discontinuation. Except for 1 participant in the nizubaglustat 3mg group who experienced a TEAE that was moderate in intensity, participants experienced only mild TEAEs. No participant experienced an SAE or died during the study.

**Table 4. Overview of Adverse Events by Disease and Treatment Group - Safety Set GM2 = Monosialoganglioside; n = Number of participants with at least one event; N = Number of participants in the Safety Set; NPC = Niemann-Pick disease Type C; TEAE = Treatment emergent adverse events. For TEAEs by worst severity, only the worst severity of each participant was counted.**

| **Number of participants (%)** | | **Nizubaglustat 9mg** | **Nizubaglustat 3mg** | **Nizubaglustat overall** | **Placebo** |
|---|---|---|---|---|---|
| | | **N=4** | **N=5** | **N=9** | **N=4** |
| **All participants, N** | | **4** | **5** | **9** | **4** |
| Any TEAE, n (%) | | 4 (100.0%) | 2 (40.0%) | 6 (66.7%) | 4 (100.0%) |
| Any drug-related TEAE, n (%) | | 2 (50.0%) | 2 (40.0%) | 4 (44.4%) | 2 (50.0%) |
| Any serious TEAE | | 0 | 0 | 0 | 0 |
| Any TEAE leading to death, n (%) | | 0 | 0 | 0 | 0 |
| Any TEAE leading to interruption of trial treatment, n (%) | | 0 | 0 | 0 | 0 |
| Any TEAE leading to discontinuation of trial treatment, n (%) | | 0 | 0 | 0 | 0 |
| TEAEs by worst severity: | | | | | |
| | Mild, n (%) | 4 (100.0%) | 1 (20.0%) | 5 (55.6%) | 4 (100.0%) |
| | Moderate, n (%) | 0 | 1 (20.0%) | 1 (11.1%) | 0 |
| | Severe, n (%) | 0 | 0 | 0 | 0 |

| **GM2 gangliosidosis, N** | | **2** | **3** | **5** | **2** |
|---|---|---|---|---|---|
| Any TEAE, n (%) | | 2 (100.0%) | 2 (66.7%) | 4 (80.0%) | 2 (100.0%) |
| Any drug-related TEAE, n (%) | | 1 (50.0%) | 2 (66.7%) | 3 (60.0%) | 0 |
| Any serious TEAE | | 0 | 0 | 0 | 0 |
| Any TEAE leading to death, n (%) | | 0 | 0 | 0 | 0 |
| Any TEAE leading to interruption of trial treatment, n (%) | | 0 | 0 | 0 | 0 |
| Any TEAE leading to discontinuation of trial treatment, n (%) | | 0 | 0 | 0 | 0 |
| TEAEs by worst severity: | | | | | |
| | Mild, n (%) | 2 (100.0%) | 1 (33.3%) | 3 (60.0%) | 2 (100.0%) |
| | Moderate, n (%) | 0 | 1 (33.3%) | 1 (20.0%) | 0 |
| | Severe, n (%) | 0 | 0 | 0 | 0 |

| **NPC, N** | | **2** | **2** | **4** | **2** |
|---|---|---|---|---|---|
| Any TEAE, n (%) | | 2 (100.0%) | 0 | 2 (50.0%) | 2 (100.0%) |
| Any drug-related TEAE, n (%) | | 1 (50.0%) | 0 | 1 (25.0%) | 2 (100.0%) |
| Any serious TEAE | | 0 | 0 | 0 | 0 |
| Any TEAE leading to death, n (%) | | 0 | 0 | 0 | 0 |
| Any TEAE leading to interruption of trial treatment, n (%) | | 0 | 0 | 0 | 0 |
| Any TEAE leading to discontinuation of trial treatment, n (%) | | 0 | 0 | 0 | 0 |
| TEAEs by worst severity: | | | | | |
| | Mild, n (%) | 2 (100.0%) | 0 | 2 (50.0%) | 2 (100.0%) |
| | Moderate, n (%) | 0 | 0 | 0 | 0 |
| | Severe, n (%) | 0 | 0 | 0 | 0 |

| | | | | | |
|---|---|---|---|---|---|
| TEAEs were events that started or worsened on or after start of trial treatment. | | | | | |

No TEAE was reported with a frequency of more than 2 participants in the study (Table 5). The system organ classes in which TEAEs were most frequently observed were Investigations (3 [33.3%] participants in the overall nizubaglustat group and 1 [25.0%] participant in the placebo group), Skin and Subcutaneous Tissue Disorders (3 [33.3%] participants in the overall nizubaglustat group and no participant in the placebo group), and Infections and Infestations (1 [11.1%] participant in the overall nizubaglustat group and 2 [50.0%] participants in the placebo group).

Among participants who received nizubaglustat, no TEAE the Investigator considered related to study drug was reported for more than 1 participant (Table 6).

A participant (5503-102) in the nizubaglustat group 3mg group had a TEAE of mildly increased blood lactate dehydrogenase (LDH) the Investigator considered related to study drug. At Screening, the participant had an LDH result below the normal range (46U/L; normal range: 125 - 220U/L). The increased LDH was first reported as a TEAE at the Week 4 visit (467U/L). At Week 12, the LDH result was 734U/L and the TEAE was ongoing. The increased LDH was considered not clinically significant.

**Table 5. Summary of All TEAEs by Primary SOC and PT by Treatment Group - Safety Set AE = Adverse event; MedDRA = Medical Dictionary for Regulatory Activities; n = Number of participants with at least one event; N = Number of participants in the Safety Set; PT = Preferred term; SOC = System organ class; TEAE = Treatment emergent adverse events.**

| **SOC, n (%)** | | **Nizubaglustat 9mg** | **Nizubaglustat 3mg** | **Nizubaglustat overall** | **Placebo** |
|---|---|---|---|---|---|
| | **PT, n (%)** | **N = 4** | **N = 5** | **N = 9** | **N = 4** |
| At least one AE | | 4 (100.0%) | 2 (40.0%) | 6 (66.7%) | 4 (100.0%) |
| Investigations | | 1 (25.0%) | 2 (40.0%) | 3 (33.3%) | 1 (25.0%) |
| | Heart rate increased | 1 (25.0%) | 0 | 1 (11.1%) | 1 (25.0%) |
| | Blood lactate dehydrogenase increased | 0 | 1 (20.0%) | 1 (11.1%) | 0 |
| | Liver function test abnormal | 0 | 1 (20.0%) | 1 (11.1%) | 0 |
| Skin and subcutaneous tissue disorders | | 3 (75.0%) | 0 | 3 (33.3%) | 0 |
| | Dermatitis | 2 (50.0%) | 0 | 2 (22.2%) | 0 |
| | Dry skin | 1 (25.0%) | 0 | 1 (11.1%) | 0 |
| Renal and urinary disorders | | 0 | 2 (40.0%) | 2 (22.2%) | 0 |
| | Urinary retention | 0 | 2 (40.0%) | 2 (22.2%) | 0 |
| Infections and infestations | | | 1 (20.0%) | 1 (11.1%) | 2 (50.0%) |
| | Sinusitis | | 1 (20.0%) | 1 (11.1%) | 1 (25.0%) |
| | Upper respiratory tract infection | 0 | 0 | 0 | 1 (25.0%) |
| Immune system disorders | | 0 | 1 (20.0%) | 1 (11.1%) | 0 |
| | Hypersensitivity | 0 | 1 (20.0%) | 1 (11.1%) | 0 |
| General disorders and administration site conditions | | 0 | 0 | 0 | 1 (25.0%) |
| | Xerosis | | | | 1 (25.0%) |
| Nervous system disorders | | 0 | 0 | 0 | 1 (25.0%) |
| | Seizure | 0 | 0 | 0 | 1 (25.0%) |
| Reproductive system and breast disorders | | 0 | 0 | 0 | 1 (25.0%) |
| | Menstruation irregular | 0 | 0 | 0 | 1 (25.0%) |
| Vascular disorders | | 0 | 0 | 0 | 1 (25.0%) |
| | Haematoma | 0 | 0 | 0 | 1 (25.0%) |

| | | | | | |
|---|---|---|---|---|---|
| A participant with >1 AE within a primary SOC was counted only once for that class. A participant with >1 AE with the same PT was counted only once for that PT. MedDRA version 27.0 was used for coding. TEAEs were events that started or worsened on or after start of trial treatment. | | | | | |

**Table 6. Summary of TEAEs Related to Study Drug by Primary SOC and PT by Treatment Group - Safety Set. AE = Adverse event; MedDRA = Medical Dictionary for Regulatory Activities; n = Number of participants with at least one event; N = Number of participants in the Safety Set; PT = Preferred term; SOC = System organ class; TEAE = Treatment emergent adverse events.**

| **SOC, n (%)** | | **Nizubaglustat 9mg** | **Nizubaglustat 3mg** | **Nizubaglustat overall** | **Placebo** |
|---|---|---|---|---|---|
| | **PT, n (%)** | **N = 4** | **N = 5** | **N = 9** | **N = 4** |
| At least one AE | | 2 (50.0%) | 2 (40.0%) | 4 (44.4%) | 2 (50.0%) |
| Investigations | | 1 (25.0%) | 1 (20.0%) | 2 (22.2%) | 1 (25.0%) |
| | Heart rate increased | 1 (25.0%) | 0 | 1 (11.1%) | 1 (25.0%) |
| | Blood lactate dehydrogenase increased | 0 | 1 (20.0%) | 1 (11.1%) | 0 |
| Immune system disorders | | 0 | 1 (20.0%) | 1 (11.1%) | 0 |
| | Hypersensitivity | 0 | 1 (20.0%) | 1 (11.1%) | 0 |
| Skin and subcutaneous tissue disorders | | 1 (25.0%) | 0 | 1 (11.1%) | 0 |
| | Dermatitis | 1 (25.0%) | 0 | 1 (11.1%) | |
| General disorders and administration site conditions | | 0 | 0 | 0 | 1 (25.0%) |
| | Xerosis | 0 | 0 | 0 | 1 (25.0%) |

| | | | | | |
|---|---|---|---|---|---|
| A participant with >1 AE within a primary SOC was counted only once for that class. A participant with >1 AE with the same PT was counted only once for that PT. MedDRA version 27.0 was used for coding. TEAEs were events that started or worsened on or after start of trial treatment. | | | | | |

## Claims

1. Nizubaglustat for use in treating a lysosomal storage disorder in a human subject suffering from the lysosomal storage disorder, wherein nizubaglustat is administered to the subject at a dose of between 3mg - 9mg total/day.

2. Nizubaglustat for use of claim 1, wherein the subject is under 21 years of age, preferably under 18 years of age, or wherein the subject is between 12 and 18 years of age.

3. Nizubaglustat for use of claim 1 or claim 2, wherein the dose is about 9 mg total/day.

4. Nizubaglustat for use of any one of claims 1-3, wherein the dose reduces a Scale for the Assessment and Rating of Ataxia (SARA) score in the subject as compared to the SARA score before administration of nizubaglustat.

5. Nizubaglustat for use of claim 4, wherein the dose reduces the SARA score by at least 1.1, preferably by at least 3, or at least 4.

6. Nizubaglustat for use of claim 4 or claim 5, wherein the dose reduces the SARA score in the subject upon continued (e.g. daily) administration for at least 2 weeks, at least 3 weeks, at least 4 weeks, at least 2 months, 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years.

7. Nizubaglustat for use of any one of claims 1-6, wherein the dose stabilises the SARA score for at least 3 months, at least 6 months, at least 1 year, at least 18 months, at least 2 years, at least 2.5 years, at least 3 years, at least 4 years, at least 5 years, at least 6 years, at least 7 years, at least 8 years, at least 9 years, or at least 10 years upon continued (e.g. daily) administration.

8. Nizubaglustat for use of any one of claims 1-7, wherein the subject suffers from seizures.

9. Nizubaglustat for use of any one of claims 1-8, wherein the dose reduces the frequency and/or duration of seizures in the subject.

10. Nizubaglustat for use of any one of claims 1-9, wherein nizubaglustat is administered to the subject for at least 3 weeks, preferably for at least 4 weeks.

11. Nizubaglustat for use of any one of claims 1-10, wherein the dose reduces the plasma GlcCer C16 levels by at least 60%, preferably at least 75%, more preferably by at least 80% as compared to the levels before administration of nizubaglustat.

12. Nizubaglustat for use of any one of claims 1-10, wherein the dose reduces:
a) the plasma GlcCer C18 levels by at least 25% as compared to the levels before administration of nizubaglustat, preferably at least 30%, more preferably by at least 35%, on daily administration for at least 4 weeks; and/or
b) the plasma GlcCer C16 levels by at least 50%, as compared to the levels before administration of nizubaglustat, preferably at least 60%, more preferably by at least 70%, on daily administration for at least 4 weeks.

13. Nizubaglustat for use of any one of claims 1-12, wherein the dose does not trigger serious or severe treatment-emergent adverse reactions in the subject.

14. Nizubaglustat for use of any one of claims 1-13, wherein the lysosomal storage disease is GM2 gangliosidosis and/or Niemann-Pick disease (e.g. type C).

15. Nizubaglustat for use of any one of claims 1-14, wherein nizubaglustat is in a crystalline form, optionally wherein the crystalline form displays a reflection, stated as a 2Θ value, at 17.2 ± 0.2°, 17.8 ± 0.2°, 21.2 ± 0.2° and/or 22.4 ± 0.2°, in an X-ray powder diffraction pattern.
